Europäisches Patentamt

European Patent Office

Office européen des brevets

(11) Veröffentlichungsnummer : **0 078 899**
**B1**

(12) ## EUROPÄISCHE PATENTSCHRIFT

(45) Veröffentlichungstag der Patentschrift :
**16.04.86**

(21) Anmeldenummer : **82108316.9**

(22) Anmeldetag : **09.09.82**

(51) Int. Cl.$^4$ : **A 61 M   5/18**, A 61 M   5/315,
A 61 B   6/00

(54) **Injektionsspritze zum aufeinanderfolgenden Einspritzen von zwei Flüssigkeiten in Blutgefässe lebender Körper.**

(30) Priorität : **11.11.81 CH 7253/81**

(43) Veröffentlichungstag der Anmeldung :
**18.05.83 Patentblatt 83/20**

(45) Bekanntmachung des Hinweises auf die Patenterteilung : **16.04.86 Patentblatt 86/16**

(84) Benannte Vertragsstaaten :
**CH DE FR GB IT LI NL SE**

(56) Entgegenhaltungen :
**CH-A-   398 446**
**CH-A-   558 742**
**FR-A- 2 194 451**
**FR-A- 2 251 339**
**US-A- 3 888 239**

(73) Patentinhaber : **CONTRAVES AG**
**Schaffhauserstrasse 580**
**CH-8052 Zürich (CH)**

(72) Erfinder : **Gähwiler, Hermann**
**Köschenrütistrasse 3**
**CH-8052 Zürich (CH)**
Erfinder : **Lorenz, Adrian, Dr.**
**Voltastrasse 33**
**CH-8044 Zürich (CH)**

Beschreibung

Die Erfindung betrifft eine Injektionsspritze zum aufeinanderfolgenden Einspritzen von zwei Flüssigkeiten, insbesondere zum Einspritzen einer Kontrastmittelflüssigkeit und einer Spülflüssigkeit in Blutgefässe lebender Körper, welche Injektionsspritze einen an dem einen Ende mit einem Befestigungsflansch und an dem anderen Ende mit einer kegelförmigen Innenwand, einer Austrittsöffnung und mit einem Katheteranschluss versehenen Zylinder sowie einen in dem Zylinder längsverschiebbar geführten Kolben umfasst.

Zum aufeinanderfolgenden Einspritzen von Medien geht aus der FR-A-2 251 339 eine Injektionsspritze hervor, die im wesentlichen einen ersten Zylinder, einen darin angeordneten, behälterartig ausgebildeten zweiten Zylinder sowie einen darin angeordneten Kolben umfasst. Der Unterschied besteht im wesentlichen darin, dass in einer ersten Ausstossphase der zweite Zylinder kolbenartig im ersten Zylinder verschiebbar ist und in einer zweiten Phase der stirnseitig mit einer Membran versehene zweite Zylinder von einer Nadel durchstossen und dadurch von dem Kolben die Flüssigkeit des zweiten Zylinders ausstossbar ist.

Aus der FR-A-2 194 451 ist eine weitere Injektionsspritze bekannt, bei welcher im Zylinder ein Kolben sowie ein kolbenartig ausgebildetes, stirnseitig mit Kreuzschlitzen versehenes Element geführt sind. Der Unterschied besteht hierbei darin, dass das Element den Zylinder lediglich in zwei beliebige Kammern unterteilt, so dass erst die vor dem Element angeordnete und anschliessend bei Oeffnung der Kreuzschlitze die dahinter angeordnete Flüssigkeit ausgestossen werden kann. Inbesondere in der Computertomographie gewinnt eine Injektionsmethode an Bedeutung, bei der unmittelbar nach der Kontrastmittelinjektion eine physiologische Kochsalzlösung eingespritzt wird. Mit einer hierfür vorgesehenen Injektionsspritze, gemäss der CH-PS-580 427, kann in einem Arbeitsgang jedoch nur eine Flüssigkeit injiziert werden.

Der Erfindung liegt die Aufgabe zugrunde, eine Injektionsspritze der eingangs genannten Gattung zu schaffen, mit der aufeinanderfolgend zwei Flüssigkeiten injiziert werden können, und welche die hohen Anforderungen bezüglich Zuverlässigkeit und Genauigkeit erfüllt, die an solche medizinische Geräte gestellt werden.

Die Aufgabe wird dadurch gelöst, dass in dem Zylinder ein von dem Kolben getrennter, jedoch von diesem direkt beauschlagter, längs verschiebbarer Behälter angeordnet ist, welcher auf der der kegelförmigen Zylinderinnenwand zugewandten Seite eine kegelförmige, mit Sollbruchstellen versehene Stirnwand aufweist, welche Sollbruchstellen nach dem Ausstossen der einen, in dem Zylinder befindlichen Flüssigkeit durch das Zusammenwirken der Stirnwand mit der Zylinderinnenwand geöffnet und dadurch ein Austreten der anderen, im Behälter befindlichen Flüssigkeit erreicht wird.

Ein Ausführungsbeispiel des Erfindungsgegenstandes wird im folgenden anhand der Zeichnung beschrieben.

Es zeigen :
Figur 1 eine teilweise im Schnitt dargestellte Injektionsspritze mit angeschlossenem Katheder,
Figur 2 eine Draufsicht auf die Stirnwand eines Behälters,
Figur 3 einen Querschnitt entlang der Linie III-III der Fig. 1,
Figur 4 ein teilweise im Schnitt dargestellter, an der Stirnwand geöffneter Behälter, eingesetzt im teilweise dargestellten Zylinder,
Figur 5 ein teilweise im Schnitt dargestellter Behälter nach einer weiteren Variante.

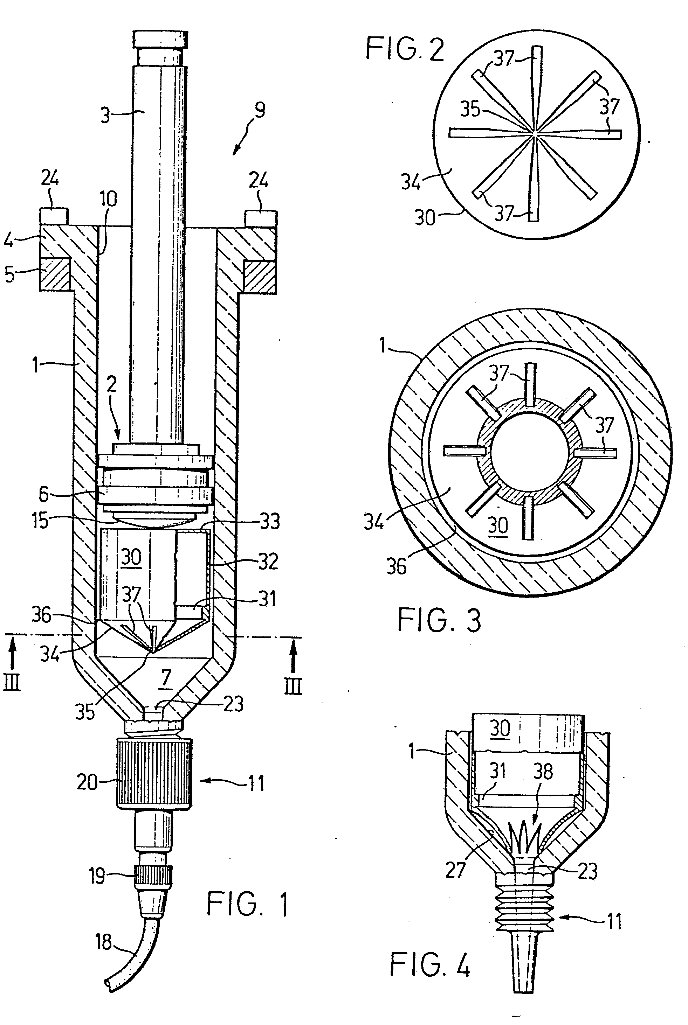

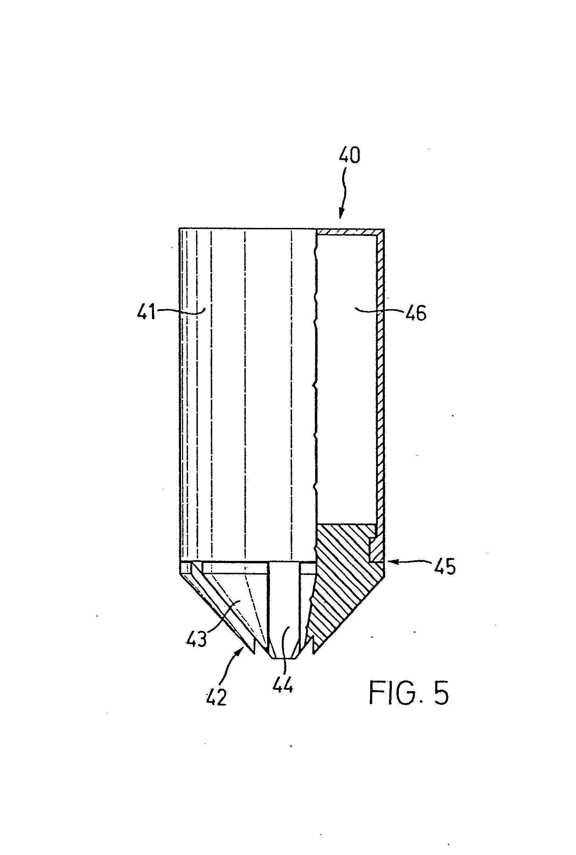

In der Fig. 1 ist eine Injektionsspritze 9 dargestellt, die aus einem Zylinder 1, einem Kolben 2 mit einem Gleitring 6 und einer Kolbenstange 3 sowie einem in den Zylinder 1 eingesetzten Behälter 30 besteht. Auf der einen Seite ist der Zylinder 1 mit einem Gewindestück 11 (Fig. 4) zur Aufnahme einer Ueberwurfmutter 20 sowie mit einer nicht bezeichneten Spitze versehen, auf welche Spitze ein mit einem Anschlussstück 19 versehener Katheter 18 aufgezogen und angeschlossen wird. Auf der anderen Seite ist ein Flansch 4, ein hinter dem Flansch 4 liegender Ring 5 sowie mindestens zwei Spann-Nocken 24 für den Anschluss der Spritze 9 an einen den Kolben 2 bewegenden Antrieb vorgesehen.

Der in den Zylinder 1 einsetzbare Behälter 30 weist eine Stirnwand 34, eine Seitenwand 32 sowie eine Rückwand 33 auf, und besteht beispielsweise aus Polyäthylen, wobei durchaus die Möglichkeit besteht, den Behälter 30 aus anderem geeigneten Material herzustellen. Die Seitenwand 32 ist zylindrisch ausgebildet und besitzt einen Aussendurchmesser, der kleiner ist als der Innendurchmesser des Zylinders 1. Zwischen der Zylinderinnenwand 10 und der Seitenwand 32 des eingesetzten Behälters 30 besteht ein zylindrischer Spalt 36, der breit genug ist, um Kontrastmittelflüssigkeit durchfliessen zu lassen, und der es erlaubt, dass der Behälter 30 im Zylinder 1 in Längsrichtung frei verschiebbar ist. Die Seitenwand 32 ist in der Nähe der Stirnwand 34 ringförmig durch einen Verstärkungsring 31 verstärkt. Die Stirnwand 34 ist kegelförmig ausgebildet und besitzt sternförmig von der Spitze 35 ausgehende Sollbruchstellen 37 (Fig. 2). Die Sollbruchstellen 37 sind Gebiete der Stirnwand, deren Wandstärken etwa 1/3 der umliegenden Wandstärken betragen (Fig. 3). Bei erhöhtem Druck im Behälter 30 brechen die Sollbruchstellen 37, worauf sich die Stirnwand 34 zu einer kronenförmigen Öffnung 38 öffnet. Ein an der Stirnwand 34 geöffneter Behälter 30 ist in der Fig. 4 dargestellt. Es ist deutlich sichtbar, wie ein Verstärkungsring 31 den Behälter 30 in seiner Position fixiert. Dadurch wird vermieden, dass sich Teile des Behälters 30 lösen und Verstopfungen verursachen.

Der Behälter 30 wird in an sich bekannter Weise, beispielsweise im Spritzverfahren, hergestellt, mit der entsprechenden Flüssigkeit gefüllt und luftdicht, beispielsweise durch Verschweissen, verschlossen.

Im folgenden soll die Verwendung der erfindungsgemässen Spritze am Beispiel einer Kontrastmittelinjektion erläutert werden. Zuerst wird ein luftfrei mit physiologischer Kochsalzlösung gefüllter Behälter 30 in den Zylinder 1 eingelegt und das Restvolumen mit der gewünschten Menge von Kontrastmittelflüssigkeit aufgefüllt. Nun wird der Kolben 2 aufgesetzt und Kontrastmittel ausgestossen. Der im Kontrastmittel schwimmende Behälter 30 befindet sich, je nach Lage des Zylinders 1, in der Nähe der Austrittsöffnung 23 (Fig. 4), oder er wird durch den Kolben zur Austrittsöffnung 23 hin geschoben. Die Kontrastmittelflüssigkeit kann im wesentlichen ohne Druckwiderstand zwischen der zylindrischen Seitenwand 32 des Behälters 30 und der Zylinderinnenwand 10 zur Austrittsöffnung 23 hin durchfliessen. Der Behälter 30 wird auch bei hohen Kolbendrücken nicht verformt, da die Flüssigkeit im Behälter im wesentlichen inkompressibel ist und sich die Kräfte auf den Behälter gegenseitig kompensieren. Ist die Kontrastmittelflüssigkeit ausgestossen, liegt der Behälter 30 in der Nähe des Verstärkungsringes 31 im Bereich eines Konischen Zylinderteils 7 auf der kegelförmig verlaufenden Innenwand 27 des Zylinders 1 auf, während die konvex, beispielsweise bombiert oder kegelförmig, ausgebildete Stirnseite 15 des Kolbens 2 die Rückwand 33 des Behälters 30 berührt. Nun wird der Kolben 2 weiter in Richtung zur Austrittsöffnung 23 hin bewegt, wodurch sich der Druck im Behälter 30 erhöht, bis die Sollbruchstellen 37 brechen und physiologische Kochsalzlösung ausgestossen wird. Der nun leicht zusammendrückbare Behälter 30 erlaubt es, durch weiteres Vorschieben des Kolbens 2 im wesentlichen alle Spülmittelflüssigkeit in der gewünschten Geschwindigkeit auszustossen.

Sowohl die Volumina der beiden Flüssigkeiten als auch der Druck im Zylinder 1 können in weiten Grenzen variiert werden. Beim aufgeführten Beispiel der Kontrastmittelinjektion beträgt der Inhalt des Behälters 30 ml, das Volumen der Kontrastmittelflüssigkeit 70 ml und der Druck im Zylinder 1 zwischen 0 und 70 bar.

Die Figur 5 zeigt einen Behälter 40 nach einer weiteren erfindungsgemässen Variante, bestehend aus einer elastischen Haube 41, deren Oeffnung von einem Frontteil 42 flüssigkeitsdichtend verschlossen ist, und dessen Innenraum 46 mit Flüssigkeit, insbesondere Spülflüssigkeit, gefüllt ist. Der konisch zugespitzte Frontteil 42 ist vorzugsweise mit Nocken 43 versehen, zwischen denen Rillen 44 verlaufen. Die Haube 41 ist vorzugsweise einstückig aus Silikongummi oder Polyäthylen gebildet, sie kann aber auch aus einem elastischen, zylindrischen Mantel mit einem, hier nicht dargestellten, aus starrem Material bestehenden Verschlussdeckel ausgebildet sein.

Die Entleerung des Behälters geschieht wie folgt : Nach dem Ausstossen der ersten Flüssigkeit wird durch Druck des Kolbens 2 auf die Haube 41 des Behälters 40 dessen Innendruck erhöht, wodurch die elastische Haube 41 erweitert wird und Flüssigkeit durch Sollöffnungsstellen 45 zwischen der Haube 41 und dem Frontteil 42 austritt und in den Rillen 44 zur Austrittsöffnung 23 fliesst. Der Behälter 40 kann weiter verwendet werden, indem er wieder mit Flüssigkeit gefüllt und der Frontteil 42 aufgesetzt wird.

**Patentansprüche**

1. Injektionsspritze zum aufeinanderfolgenden Einspritzen von zwei Flüssigkeiten, insbesondere zum Einspritzen einer Kontrastmittelflüssigkeit und einer Spülflüssigkeit in Blutgefässe lebender Körper, welche Injektionsspritze (9) einen an dem einen Ende mit einem Befestigungsflansch (4) und an dem anderen Ende mit einer kegelförmigen Innenwand (27), einer Austrittsöffnung (23) und mit einem Katheteranschluss (11) versehenen Zylinder (1) sowie einen in dem Zylinder längsverschiebbar geführten Kolben (2) umfasst, dadurch gekennzeichnet, dass in dem Zylinder (1) ein von dem Kolben (2) getrennter, jedoch von diesem direkt beaufschlagter, längs verschiebbarer Behälter (30, 40) angeordnet ist, welcher auf der der kegelförmigen Zylinderinnenwand (27) zugewandten Seite eine kegelförmige, mit Sollbruchstellen (37, 45) versehene Stirnwand (34, 42) aufweist, welche Sollbruchstellen nach dem Ausstossen der einen, in dem Zylinder (1) befindlichen Flüssigkeit durch das Zusammenwirken der Stirnwand (34, 42) mit der Zylinderinnenwand (27) geöffnet und dadurch ein Austreten der anderen, im Behälter (30, 40) befindlichen Flüssigkeit erreicht wird.

2. Injektionsspritze nach Anspruch 1, dadurch gekennzeichnet, dass der Behälter (40) eine zylindrische Haube (41) aus elastischem Material hat und auf der der kegelförmigen Zylinderinnenwand (27) zugewandten Seite von einem die Stirnwand bildenden kegelförmigen Frontteil (42) dichtend verschlossen ist, und dass am Uebergang von der Haube (41) zum Frontteil (42) die Sollöffnungsstellen (45) und an der Aussenseite des Frontteils (42) strahlenförmig verlaufende Rillen (44) vorgesehen sind.

3. Injektionsspritze nach Anspruch 1, dadurch gekennzeichnet, dass der Behälter (30) am Uebergang seiner zylindrischen Seitenwand (32) zu der kegelförmigen Stirnwand (34) eine ringförmige Verstärkung (31) aufweist.

4. Injektionsspritze nach Anspruch 1, dadurch gekennzeichnet, dass die eine geringere Wandstärke aufweisenden Sollöffnungsstellen (37) des Behälters (30) von der Spitze (35) bis zu der zylindrischen Seitenwand (32) strahlenförmig an der kegelförmigen Stirnwand (34) angeordnet sind.

5. Injektionsspritze nach einem der Ansprüche 1-4, dadurch gekennzeichnet, dass der Zylinder (1) für den ersten Injektionsvorgang eine Kontrastmittelflüssigkeit und der Behälter (30, 40) für den anschliessenden Zweiten Injektionsvorgang eine physiologische Kochsalzlösung enthält.

## Claims

1. Syringe for the sequential injection of two fluids, especially for the injection of a contrast medium fluid and a rinsing fluid into the blood vessels of living bodies, which syringe (9) comprises a cylinder (1) provided at one end with a fastening flange (4) and at the other end with a conical inner wall (27), an exit opening (23) and a catheter connection (11), as well as a piston (2) which can be guided displaceably longitudinally in the cylinder, characterized in that, in the cylinder (1), a longitudinally displaceable container (30, 40), separate from the piston (2) yet acted upon directly by it, is arranged, which comprises on the side facing the conical cylinder inner wall (27), a conical front wall (34, 42) provided with predetermined breaking points (37, 45), which predetermined breaking points, after the expulsion of the one fluid located in the cylinder (1), is opened through the combined action of the front wall (34, 42) with the cylinder inner wall (27) and thereby an extravasation of the other fluid, located in the container (30, 40), is achieved.

2. Syringe according to claim 1, characterized in that the container (40) has a cylindrical hood (41) of elastic material and is sealingly closed, on the side facing the conical cylinder inner wall (27), by a conical front part (42) forming the front wall and in that, at the transition from the hood (41) to the front part (42) the predetermined opening points (45) are provided and, on the outer side of the front part (42), grooves (44) running radially are provided.

3. Syringe according to claim 1, characterized in that the container (30) comprises, at the transition of its cylindrical side wall (32) to the conical front wall (34) an annular strengthening piece (31).

4. Syringe according to claim 1, characterized in that the predetermined opening points (37), having less wall thickness, of the container (30), are arranged from the tip (35) up to the cylindrical side wall (32), radially on the conical front wall (34).

5. Syringe according to one of the claims 1-4, characterized in that the cylinder (1) contains, for the first injection process, a contrast medium fluid and the container (30, 40) contains, for the sequential second injection process, a physiological saline solution.

## Revendications

1. Seringue destinée à l'injection consécutive de deux fluides dans les vaisseaux sanguins de corps vivants, notamment pour l'injection d'un liquide de contraste et d'un liquide de rinçage dans les vaisseaux sanguins de corps vivants, cette seringue (9) ayant un cylindre (1) dont une extrémité est munie d'une bride de fixation (4) et dont l'autre extrémité comporte une paroi intérieure (27) en forme de cône et un orifice de sortie (23) ainsi qu'un raccord de cathéter (11) et un piston (2) guidé coulissant longitudinalement dans le cylindre, caractérisée en ce que dans le cylindre (1) il est prévu un récipient (30, 40) coulissant longitudinalement, distinct du piston (2) mais susceptible d'être sollicité directement par celui-ci, récipient qui comporte sur le côté tourné vers la paroi intérieure conique (27) du cylindre, une paroi frontale (34, 42) munie de zones de rupture de consigne (37, 45), ces zones s'ouvrant par l'expulsion d'un liquide se trouvant dans le cylindre (1) par la coopération de la paroi frontale (34, 42) et de la paroi intérieure (27) du cylindre, et permettant l'émission de l'autre liquide se trouvant dans le réservoir (30, 40).

2. Seringue selon la revendication 1, caractérisé en ce que le réservoir (40) a une coiffe cylindrique (41) en matière élastique et le côté tourné vers la paroi interne conique (27) du cylindre est fermé de façon étanche par une partie frontale (42) conique formant la paroi frontale, et en ce qu'à la transition entre la coiffe (41) et la paroi frontale (42) sont prévues des zones d'ouverture de consigne (45) et sur le côté extérieur de la partie frontale (42) sont prévues des nervures (44) radiales.

3. Seringue selon la revendication 1, caractérisée en ce que le réservoir (30) présente un renforcement annulaire (31) à sa transition entre sa paroi latérale cylindrique (32) et sa paroi frontale (34) conique.

4. Seringue selon la revendication 1, caractérisée en ce que les zones de rupture de consigne (37) du réservoir (30) et qui ont une faible épaisseur de paroi, vont de la pointe (35) jusqu'à la paroi latérale (32) cylindrique, de façon radiale sur la paroi frontale (34) conique.

5. Seringue selon l'une des revendications 1 à 4, caractérisée en ce que le cylindre (1) contient un liquide de contraste pour la première phase d'injection et le réservoir (30, 40) contient une solution physiologique de sel de cuisine pour la seconde phase d'injection, consécutive.

0 078 899

FIG. 1

FIG. 2

FIG. 3

FIG. 4

FIG. 5